**Europäisches Patentamt**

**European Patent Office**

**Office européen des brevets**

(19)

(11) Numéro de publication: **0 031 278**
**A1**

(12) ## DEMANDE DE BREVET EUROPEEN

(21) Numéro de dépôt: **80401777.0**

(22) Date de dépôt: **11.12.80**

(51) Int. Cl.³: **G 02 B 5/28**
**G 02 B 5/22, G 02 C 7/10**

(30) Priorité: **19.12.79 FR 7931037**

(43) Date de publication de la demande:
**01.07.81 Bulletin 81/26**

(84) Etats Contractants Désignés:
**DE GB SE**

(71) Demandeur: **ETAT-FRANCAIS représenté par le DELEGUE GENERAL POUR L'ARMEMENT**
**Bureau des Brevets et Inventions de la Délégation Générale pour l'Armement 14, rue Saint-Dominique F-75997 Paris Armées(FR)**

(72) Inventeur: **Farges, Guy**
**7, Boulevard de Stalingrad**
**F-92320 Chatillon(FR)**

(54) **Filtre optique interférentiel de protection contre les radiations infrarouges et application.**

(57) L'invention concerne un filtre optique interférentiel de protection contre les radiations infrarouges dont la bande passante est située dans le visible et centrée sur le maximum de sensibilité de l'oeil.

Ce filtre comprend un support au moins partiellement transparent dans la bande visible sur une face duquel est déposé le filtre proprement dit constitué de n éléments filtrants comportant chacun une couche de metal, l'argent par exemple, interposée entre deux couches d'un matériau diélectrique transparent à haut indice de réfraction par exemple le sulfure de zinc, n étant un nombre entier supérieur ou égal à 3. L'épaisseur des couches métalliques est de l'ordre de 20 nm et celle des couches diélectriques de l'ordre de 30 à 45 nm.

Ce filtre est apte à la réalisation des dispositifs pour la protection de l'oeil par exemple contre les radiations émises par des lasers au néodyme.

Croydon Printing Company Ltd.

FILTRE OPTIQUE INTERFERENTIEL DE PROTECTION CONTRE LES RADIATIONS INFRAROUGES ET APPLICATION.

Le secteur technique de la présente invention est celui des filtres optiques utilisés pour atténuer fortement les radiations infrarouges du spectre électromagnétique tout en transmettant les radiations visibles.

Il est bien connu que le phénomène de la vision est assuré par les seules radiations du spectre visible, c'est à dire les radiations électromagnétiques ayant des longueurs d'onde comprises entre 400 et 700 nm. Ces radiations sont sans danger pour l'oeil si leur intensité n'est pas trop importante, contrairement à celles des domaines ultraviolet et infrarouge qui peuvent entraîner de graves lésions, même à des doses relativement faibles. Plus particulièrement, lorsqu'on désire se protéger contre l'émission d'un laser au néodyme, le filtre de protection idéal devrait présenter un facteur de transmission maximum sur toute l'étendue du domaine visible et atténuer considérablement la radiation $\lambda$ = 1060 nm. L'atténuation peut être réalisée soit par absorption, soit par réflexion de la radiation nuisible. On va donc s'intéresser plus spécialement à l'élimination de la radiation 1060 nm, sans que cela soit considéré comme une limitation des applications de la présente invention.

La protection des yeux contre l'émission d'un laser au néodyme est le plus souvent réalisée par l'utilisation de verres minéraux ou organiques teintés dans la masse. Un exemple de verre minéral utilisé pour la protection infrarouge est le verre type KG 3, fabriqué par la Société allemande SCHOTT. Sous une épaisseur de 2 mm ce verre présente une densité optique de l'ordre de 3 pour $\lambda$ = 1060 nm et un facteur de transmission moyen dans le visible d'environ 70%. Une densité optique supérieure dans l'infrarouge est obtenue en augmentant l'épaisseur du verre. Il est à noter que ce filtre atténue l'ensemble du spectre infrarouge.

Un exemple de verre organique a été proposé par la Société américaine GLENDALE OPTICAL CORPORATION INC; il permet d'obtenir une densité optique très élevée ( $\simeq$ 30) pour $\lambda$ = 1060 nm. Toutefois le facteur de transmission maximum dans le visible est seulement de l'ordre de 20% et la largeur de la bande passante est environ de 60 nm. La vision à travers ce filtre est donc affaiblie et de plus la perception des couleurs est extrêmement déformée.

Outre ces dispositifs, d'autres solutions ont été proposées pour atténuer les radiations infrarouges et notamment l'utilisation de supports en verre ou en matière plastique recouverts d'une ou plusieurs couches minces métalliques, en particulier d'or ou de cuivre.

Par exemple le brevet américain 3 118 781 propose un filtre comprenant un support par exemple en Mylar recouvert par une couche d'or protégée par une couche externe de résine. Ce genre de filtre, bien que donnant satisfaction pour certaines utilisations telle que la protection des soudeurs, ne peut convenir pour la protection laser à $\lambda$ = 1060 nm. En effet, la densité optique pour cette longueur d'onde est très insuffisante et le domaine visible est considérablement atténué; de plus la surface interne de ce type de filtre étant très réfléchissante; l'oeil reçoit la majeure partie de la lumière arrivant par l'arrière, d'où un inconfort visuel intolérable.

La solution consistant à utiliser des filtres complexes composés d'un support recouvert de plusieurs couches minces, tel que le propose le brevet français 2 003 177, présente l'avantage d'avoir une surface interne ne réfléchissant pas la lumière visible. Ils possèdent cependant l'inconvénient des filtres à couches métalliques, d'atténuer exagérément le domaine visible sans assurer une élimination franche de la radiation $\lambda$ = 1060 nm.

Dans le brevet français 2 276 601, on a proposé un filtre de bande destiné à la protection de la vue contre l'éclair luminothermique d'une explosion nucléaire. Ce filtre est composé d'un support recouvert d'un ensemble de couches métalliques et diélectriques alternées. Si ce filtre atténue fortement la transmission des domaines ultraviolet et infrarouge tout en assurant une perception fidèle des couleurs, il possède l'inconvénient d'atténuer notablement le domaine visible. A titre d'exemple, un filtre possédant une densité optique de l'ordre de 4,5 pour $\lambda$ = 1060 nm a un facteur de transmission de l'ordre de 10% pour $\lambda$ = 550 nm.

Les empilements constitués de matériaux diélectriques alternativement à haut indice et à bas indice de réfraction offrent la possibilité d'obtenir un facteur de transmission très élevé dans le visible tant en atténuant la longueur d'onde 1060 nm. Toutefois l'obtention d'une densité optique élevée à cette longueur d'onde nécessite de recourir a un nombre important de couches, de l'ordre d'une vingtaine.

Dans le brevet français 2 106 431 on a proposé un filtre interférentiel destiné à transmettre la lumière visible et à réfléchir le domaine infrarouge. Ce filtre est composé par exemple d'un support sur lequel sont déposées dans l'ordre une couche de diélectrique à base d'alumine, une couche métallique, une couche diélectrique à base de sulfure de zinc, une couche métallique et enfin une couche diélectrique. Dans la revue "APPLIED OPTICS" vol 15, No 4, avril 1976, on décrit un filtre utilisable contre la transmission des radiations infrarouges et ultraviolettes comprenant un support, une couche de dioxyde de titane, une couche métallique (l'argent ) et une couche de dioxyde de titane. La transmission moyenne dans le visible est de l'ordre de 70 à 80% mais la densité optique pour $\lambda$ = 1060 nm est seulement de l'ordre de l'unité. Il n'est pas question d'utiliser de tels filtres pour la protection contre l'émission d'un laser au néodyme.

Dans le brevet américain (redélivré) 27 473, on a proposé un filtre pour la réalisation de verres de lunettes filtrant l'infrarouge et l'ultraviolet composé d'un support transparent en verre sur lequel sont déposées une couche d'un diélectrique (dioxyde de titane par exemple) une couche métallique (l'argent ou l'or par exemple) et enfin une couche d'un élément antiréfléchissant. Cependant la transmission est très faible au voisinage 510 nm.

La présente invention a pour but de fournir un filtre optique destiné à assurer une protection des yeux contre les radiations infrarouges tout en pertubant aussi peu que possible la vision et notamment la perception des couleurs. Elle a encore pour but d'assurer cette protection même en présence de flux lumineux intense à la longueur d'onde $\lambda$ = 1060 nm.

L'invention a donc pour objet un filtre optique interférentiel de protection contre les radiations infrarouges dont la bande passante est située dans le visible et centrée sur le maximum de sensibilité de l'oeil, comprenant un support au moins particllement transparent dans ladite bande sur une face duquel est déposé un filtre optique, caractérisé en ce que le filtre est constitué de n éléments filtrants comportant chacun une couche de métal interposée entre deux couches d'un matériau diélectrique transparent à haut indice de réfraction, n étant un nombre entier supérieur ou égal à 3.

L'épaisseur des couches métalliques est sensiblement supérieure ou égale à 20 nm et l'épaisseur des couches du matériau diélectrique comprise entre environ 30 à 45 nm.

Avantageusement les couches métalliques sont choisies dans le groupe : aluminium, cuivre, rhodium, platine, or, argent et le matériau diélectrique dans le groupe des oxydes de : bismuth, plomb, étain, indium, aluminium, silicium, terres rares; dioxydes de : titane, zirconium; pentoxydes de : niobium, tantale et le sulfure de zinc.

La couche métallique est en argent et la couche de matériau diélectrique en sulfure de zinc.

Préférentiellement, dans un mode de réalisation destiné à la protection contre les radiations émises par les lasers au néodyme avec une densité optique de l'ordre de 4 pour $\lambda$ = 1060 nm, n est égal à 4, l'épaisseur de la couche de diélectrique intermédiaire en sulfure de zinc est de l'ordre de 70 nm et l'épaisseur des couches de diélectrique extrèmes est de l'ordre de 40 nm.

Dans un autre mode de réalisation destiné à la protection contre les radiations émises par les lasers un néodyme avec une densité optique de l'ordre de 5,5 pour $\lambda$ = 1060 nm, n est égal à 5, l'épaisseur de la couche de diélectrique intermédiaire en sulfure de zinc est de l'ordre de 40 nm.

Le filtre optique selon l'invention est apte à la réalisation de dispositifs pour la protection de l'oeil humain.

Un premier résultat procuré par le filtre selon l'invention réside dans le fait que la densité optique obtenue est élevée dans le domaine des radiations infrarouges, tout en procurant un facteur de transmission de l'ordre de 10% le domaine visible.

Un autre résultat réside dans le fait que la bande passante du filtre selon l'invention est centrée sur le maximum de sensibilité de l'oeil ($\lambda$ = 550 nm) et sa largeur de bande est de l'ordre de 220 nm, ce qui perturbe peu la perception des couleurs.

Un autre résultat du filtre selon l'invention est d'être réalisable sur n'importe quel type de support optique et en particulier sur des supports bombés de grande surface. Il présente l'avantage de résister au test du ruban adhésif et de satisfaire aux essais climatiques et d'environnement en vigueur pour ce type de matériel.

Les propriétés optiques du filtre selon l'invention sont homogènes et varient peu en fonction de l'angle d'incidence d'un faisceau laser.

Le filtre de protection objet de l'invention comprend donc un support transparent ou semi-transparent sur une face duquel sont déposés les éléments filtrants. Tous les supports habituellement utilisés dans ce domaine technique peuvent être utilisés; il suffit qu'ils transmettent bien l'énergie visible. A titre d'exemple, on peut utiliser un verre minéral ou une matière plastique. L'épaisseur de ce support n'est pas critique, il faut cependant qu'il assure la rigidité mécanique de l'ensemble.

Les couches métalliques peuvent être en un ou plusieurs des métaux usuellement employés en optique interférentielle tels que l'aluminium, le cuivre, le rhodium, le platine, mais on utilisera avantageusement l'or ou mieux encore l'argent. Les couches diélectriques sont également constituées d'un ou plusieurs des matériaux utilisés couramment en optique interférentielle tels que l'oxyde de bismuth, l'oxyde de plomb, l'oxyde d'étain, l'oxyde d'indium, le dioxyde de titane, le dioxyde de zirconium, le pent-oxyde de niobium, le pentoxyde de tantale, l'oxyde d'aluminium, les oxydes de silicium, le sulfure de zinc, les oxydes des terres rares. Cependant, on utilisera avantageusement, pour l'optimisation de la valeur du facteur de transmission maximum dans le visible et de la largeur de la bande passante, les matériaux à haut indice de réfraction, c'est à dire le dioxyde de titane et le sulfure de zinc. En effet, pour un métal donné, or ou argent de préférence, on a constaté que le facteur de contraste de l'élément optique est d'autant plus élevé que l'indice de réfraction du matériau transparent est lui-même plus élevé.

Il est connu que l'épaisseur des couches diélectriques détermine la position de la bande passante dans le spectre visible. Cependant on a découvert qu'il existe, en première approximation, un rapport optimal entre les épaisseurs des couches métalliques et diélectriques. Selon l'invention, les épaisseurs optiques des couches diélectriques sont choisies de façon à obtenir un traitement antiréflechissant au maximum pour les couches de métal dans le visible. On obtient ainsi un facteur de transmission maximum dans ce domaine. Mais d'autre part plus les couches de métal seront épaisses et plus la densité optique à $\lambda$ = 1060 nm et d'une manière générale dans l'infrarouge sera élevée. De ce compromis résulte l'invention, qui utilise des couches de métal dont l'épaisseur est au moins égale à 20 nm.

Pour réaliser un filtre selon l'invention on peut opérer de la manière suivante. On utilise par exemple la technique d'évaporation sous vide ou de pulvérisation cathodique ou des techniques équivalentes pour déposer les couches successives de métal et de diélectrique. Le support est disposé sur un ensemble tournant et les sources d'évaporation des métaux et des diélectriques sont placées au voisinage de cet ensemble. L'épaisseur des différentes couches est contrôlée de manière connue par le repérage du facteur de transmission du filtre pendant sa fabrication.

L'invention sera mieux comprise à la lumière de modes de réalisation particuliers donnés à titre indicatif sans aucun caractère limitatif en relation avec les dessins :

- la figure 1 représente une coupe transversale d'un mode de réalisation dans lequel n = 4.
- la figure 2 représente également une coupe transversale d'un mode de réalisation selon lequel n = 5;

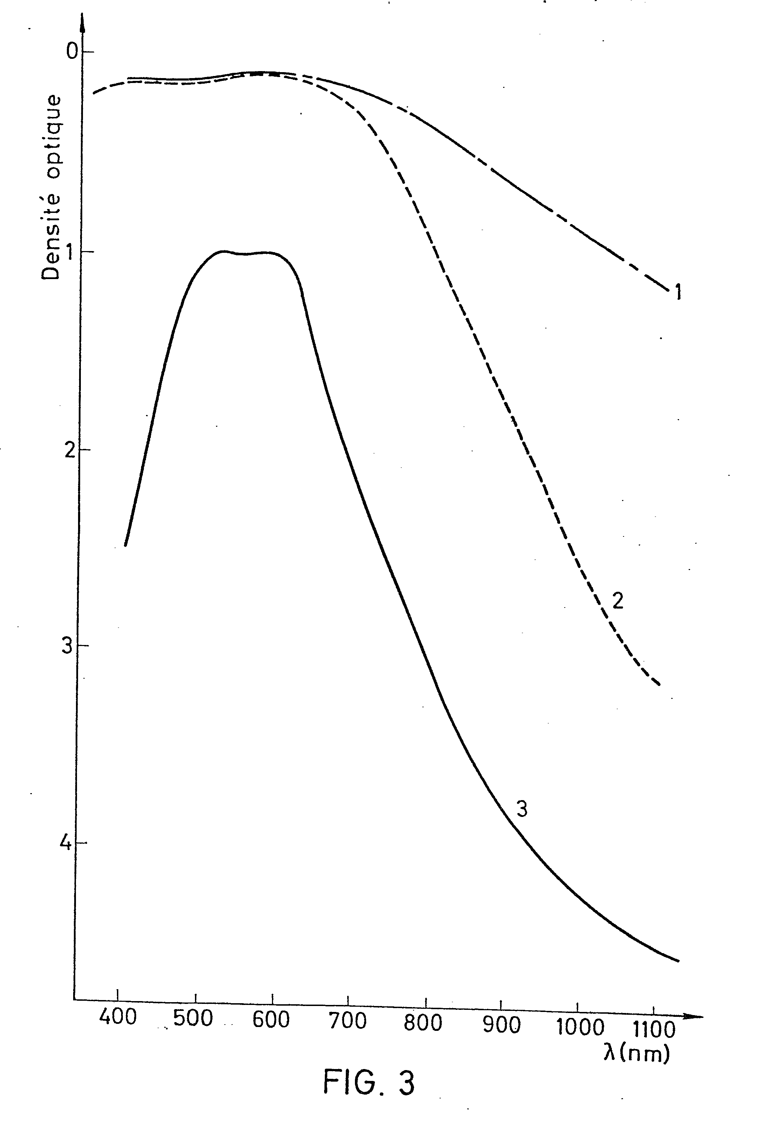

- la figure 3 représente la variation de la densité optique d'un certain nombre de filtres de l'art antérieur en fonction de la longueur d'onde.
- la figure 4 représente la variation de la densité optique de deux filtres selon l'invention en fonction de la longueur d'onde.

On prépare les filtres selon l'invention par la technique d'évaporation sous vide dans un évaporateur BALZERS type BAK 550 fabriqué par la Société BALZERS. Les supports à traiter, lames de verre minéral transparent d'indice de réfraction n = 1,52 ou disques en matériau plastique tel que du polycarbonate, sont disposés sur un dôme tournant situé à 600 mm de la base de celle-ci. Les sources d'évaporation situées sur un arc de cercle de diamètre 400 mm, à une hauteur de 150 mm de la base de l'évaporateur, sont 2 nacelles l'une en tungstène et l'autre en molybdène. La nacelle en tungstène contient de l'or ou de l'argent la nacelle en molybdène, du sulfure de zinc. Un dispositif de mesure de l'épaisseur des couches permet de contrôler en permanence le facteur de transmission du filtre pendant son élaboration.

Après avoir effectué le vide dans l'évaporateur jusqu'à une pression de quelques $10^{-2}$ Torr, on produit pendant 10 nanominutes une décharge luminescente destinée à parfaire le nettoyage des supports par bombardement ionique. Puis on poursuit le pompage jusqu'à l'obtention d'une pression de $1 \times 10^{-6}$ Torr. On dépose alors les différentes couches des matériaux sans interrompre le vide, en chauffant alternativement et successivement les nacelles aux températures d'évaporation de l'argent ou de sublimation du sulfure de zinc. Lorsque l'épaisseur désirée pour chaque couche est atteinte un écran interposé entre les sources d'évaporation et les supports vient interrompre rapidement le dépôt.

EXEMPLE 1 .

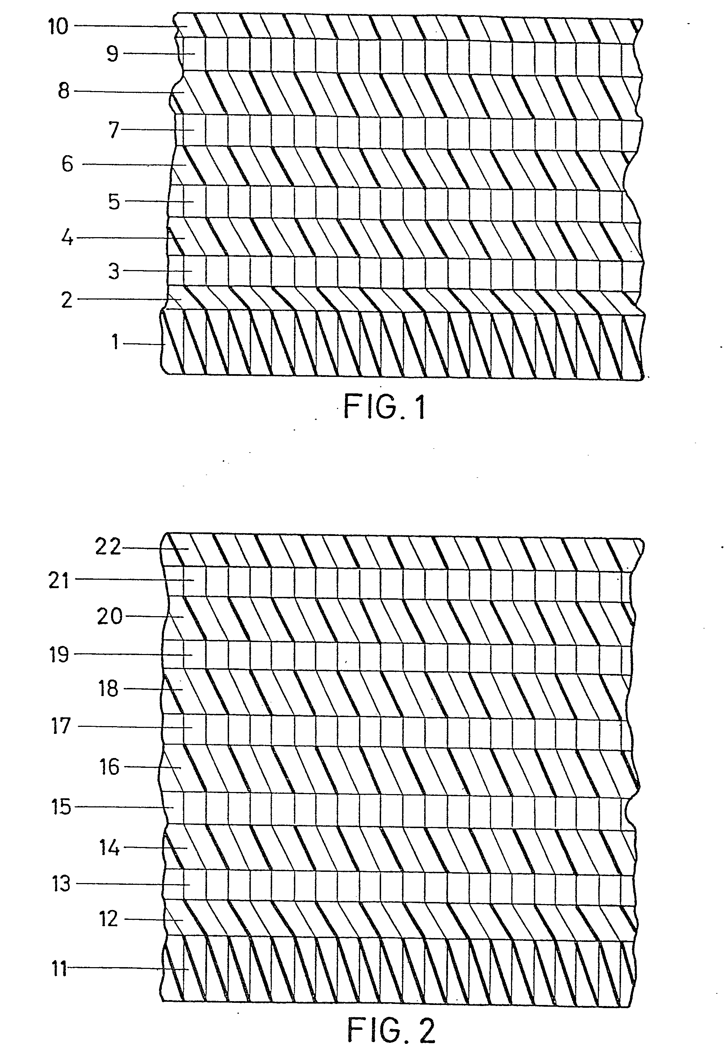

On réalise comme indiqué précédemment le filtre schématisé sur la figure 1; sur le support 1 en verre d'indice de réfraction n = 1,52, on dépose alternativement les couches de sulfure de zinc 2,4,6,8,10 et les couches d'argent 3,5,7,9 avec les épaisseurs suivantes :

| Numéro de la couche | Indice du matériau ou matériau | Epaisseur en nm |
|---|---|---|
| 10 | 2,3 | 40 |
| 9 | Ag | 20 |
| 8 | 2,3 | 73 |
| 7 | Ag | 23 |
| 6 | 2,3 | 70 |
| 5 | Ag | 23 |
| 4 | 2,3 | 73 |
| 3 | Ag | 20 |
| 2 | 2,3 | 42 |

Sur la figure 1 la courbe 1 représente la densité optique d'un filtre selon le brevet français 2 106 431, la courbe 2 représente la densité optique d'un verre organique utilisé dans l'art antérieur et la courbe 3 celle d'un filtre selon le brevet français 2 276 601.

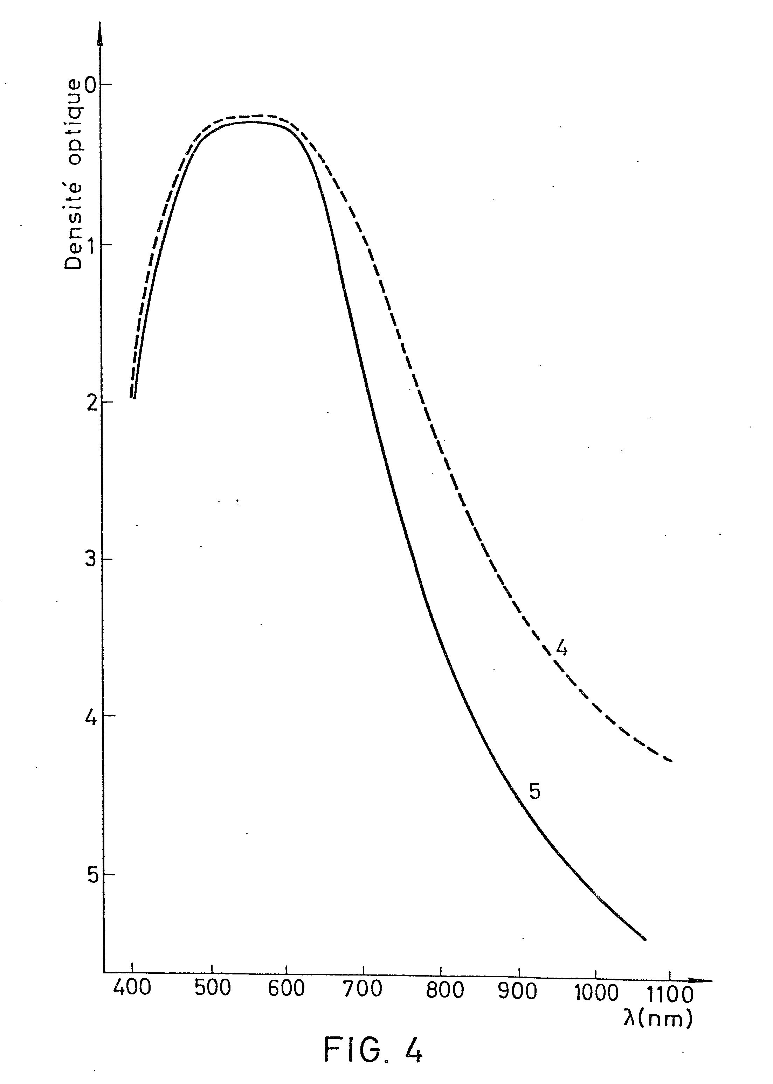

Sur la figure 4 la courbe 4 représente la densité optique du filtre précédemment préparé et on voit qu'au voisinage de $\lambda$ = 1060 nm celui-ci présente une densité de l'ordre de 4, valeur qui est nettement supérieure aux valeurs antérieurement obtenues selon les courbes 1 et 2 et pratiquement du même ordre de grandeur que celle obtenue selon la courbe 3.

EXEMPLE 2.

On réalise de la même manière, le filtre schématisé sur la figure 2 en déposant sur le support 11 les couches de sulfure de zinc 12,14,16, 18,20,22 et les couches d'argent 13,15,17,19,21 avec les épaisseurs suivantes :

| Numéro de la couche | Indice du matériau ou matériau | Epaisseur en nm |
|---|---|---|
| 22 | 2,3 | 40 |
| 21 | Ag | 20 |
| 20 | 2,3 | 73 |
| 19 | Ag | 23 |
| 18 | 2,3 | 70 |
| 17 | Ag | 24 |
| 16 | 2,3 | 70 |
| 15 | Ag | 23 |
| 14 | 2,3 | 73 |
| 13 | Ag | 20 |
| 12 | 2,3 | 42 |

**0031278**

La courbe 5 de la figure 4 représente la variation de la densité optique d'un tel filtre. On voit qu'au voisinage de $\lambda = 1060$ nm ce filtre présente une densité optique supérieure à 5,5. Un filtre de ce type est particulièrement bien adapté pour la protection contre l'émission d'un laser au néodyme.

Par rapport au mode relatif à la figure 1 ce filtre possède une couche métallique et une couche diélectrique supplémentaires. Ces deux couches ont pour effet d'accroître d'une unité la valeur de la densité optique à $\lambda = 1060$ nm sans altérer notablement la transmission du domaine visible qui reste très élevé.

Diverses modifications à la portée de l'homme de l'art peuvent être apportées au filtre selon l'invention. C'est ainsi que dans le but de parfaire la transmission dans le visible il est possible d'interposer une couche d'un matériau à bas indice de réfraction de préférence entre le support et la première couche diélectrique.

**0031278**

<div align="center">REVENDICATIONS</div>

1 - Filtre optique interférentiel de protection contre les radiations infrarouges dont la bande passante est située dans le visible et centrée sur le maximum de sensibilité de l'oeil, comprenant un support au moins partiellement transparent dans ladite bande sur une face duquel est déposé un filtre optique, caractérisé en ce que le filtre est constitué de n éléments filtrants comportant chacun une couche de métal interposée entre deux couches d'un matériau diélectrique transparent à haut indice de réfraction, n étant un nombre entier supérieur ou égal à 3.

2 - Filtre optique selon la revendication 1, caractérisé en ce que l'épaisseur des couches métalliques est sensiblement supérieure ou égale à 20 nm et l'épaisseur des couches du matériau diélectrique comprise entre environ 30 à 45 nm.

3 - Filtre optique selon la revendication 1 ou 2, caractérisé en ce que les couches métalliques sont choisies dans le groupe : aluminium, cuivre, rhodium, platine, or, argent et le matériau diélectrique dans le groupe des oxydes de : bismuth, plomb, étain, indium, aluminium, silicium, terres rares; dioxydes de : titane, zirconium; pentoxydes de : niobium, tantale et le sulfure de zinc.

4 - Filtre optique selon la revendication 3, caractérisé en ce que la couche métallique est en argent et la couche de matériau diélectrique en sulfure de zinc.

5 - Filtre optique selon la revendication 4 destiné à la protection contre les radiations émises par des lasers au néodyme, caractérisé en ce que n = 4, l'épaisseur des couches de sulfure de zinc intermédiaires étant de l'ordre de 70 nm et l'épaisseur des couches de sulfure de zinc extrêmes est de l'ordre de 40 nm.

6 - Filtre optique selon la revendication 5 destiné à la protection contre les radiations émises par les lasers en néodyme, caractérisé en ce que n = 5, l'épaisseur des couches de sulfure de zinc intermédiaires étant de l'ordre de 70 nm et l'épaisseur des couches de sulfure de zinc extrêmes est de l'ordre de 40 nm.

0031278

7 - Application des filtres selon l'une quelconque des revendications 1 à 4 à la réalisation de dispositifs pour la protection de l'oeil humain.

0031278

FIG. 1

FIG. 2

FIG. 3

PL. III/3

0031278

FIG. 4

Office européen
des brevets

**RAPPORT DE RECHERCHE EUROPEENNE**

Numéro de la demande
EP 80 40 1777

| | | CLASSEMENT DE LA DEMANDE (Int. Cl. 3) |
|---|---|---|
| **DOCUMENTS CONSIDERES COMME PERTINENTS** | | G 02 B 5/28<br>5/22<br>G 02 C 7/10 |

| Catégorie | Citation du document avec indication, en cas de besoin, des parties pertinentes | Revendication concernée |
|---|---|---|
| | FR - A - 1 513 724 (GLAVERBEL)<br>  * Page 5, colonne de gauche,<br>   ligne 36 à colonne de droite,<br>   ligne 57 *<br><br>      -- | 1,3 |
| DA | FR - A - 2 276 601 (ETAT FRAN-<br>CAIS REPRESENTE PAR LE DELEGUE<br>MINISTERIEL POUR L'ARMEMENT)<br>  * En entier *<br><br>      ----- | 1 |

**DOMAINES TECHNIQUES RECHERCHES (Int. Cl. 3)**

G 02 B 5/28
5/22
G 02 C 7/10

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

Le présent rapport de recherche a été établi pour toutes les revendications

| Lieu de la recherche | Date d'achèvement de la recherche | Examinateur |
|---|---|---|
| La Haye | 18-03-1981 | TREVETIN |

OEB Form 1503.1   06.78